# EUROPEAN PATENT SPECIFICATION

(11) **EP 2 131 755 B1**
(45) Date of publication and mention of the grant of the patent: **05.10.2011**
(21) Application number: 08731061.1
(22) Date of filing: 29.02.2008
(51) Int. Cl.: A61B 17/12

(54) **System for delivering a detachable implantable device**
System zur Abgabe eines lösbaren implantierbaren Produkts
Système permettant de fournir un dispositif implantable détachable

(30) Priority: 08.03.2007 US 893720 P
(43) Date of publication of application: 16.12.2009
(73) Proprietor: Boston Scientific Limited, Barbados, West Indies (BB)
(72) Inventor: BUISER, Marcia, Marlborough, MA 01752 (US); NARDONE, Christopher, N. Chelmsford, Massachusetts 01863 (US); ELLIOTT, Christopher, Hopkinton, MA 01748 (US)
(74) Representative: Besnard, Christophe Laurent
(86) International application number: PCT/US2008/055418
(87) International publication number: WO 2008/109394

(56) References cited:
- EP-A- 0 829 236
- WO-A-93/11719
- WO-A-93/11825

## Description

### BACKGROUND OF THE INVENTION

The endovascular treatment of a variety of maladies throughout the body is an increasingly more important form of therapy. One such procedure uses embolizing coils to occlude a target site by posing a physical barrier to blood flow and/or by promoting thrombus formation at the site. Such treatments can be useful where it is desired to reduce vascularization, including treatments for aneurysms and cancer.

Coils have typically been placed at the desired site within the vasculature using a catheter and a pusher. As a first step, a flexible, small diameter catheter can be guided to the target site through the use of guidewires or by flow-directed means such as balloons placed at the distal end of the catheter. Once the site has been reached, the catheter lumen is cleared by removing the guidewire (if a guidewire has been used), and the coil is placed into the proximal open end of the catheter and advanced through the catheter with a pusher. Pushers are wires having a distal end that is adapted to engage and push the coil through the catheter lumen as the pusher is advanced through the catheter. When the coil reaches the distal end of the catheter, it is discharged from the catheter by the pusher into the vascular site.

Several techniques have been developed to enable more accurate placement of coils within a vessel. In one technique the coil is bonded via a metal-to-metal joint to the distal end of the pusher. The pusher and coil are made of dissimilar metals. The coil-carrying pusher is advanced through the catheter to the site and a small electrical current is passed through the pusher-coil assembly. The current causes the joint between the pusher and the coil to be severed via electrolysis. The pusher may then be retracted leaving the detached coil at an exact position within the vessel. In addition to enabling accurate coil placement, the electric current may facilitate thrombus formation at the coil site. A perceived disadvantage of this method is that the electrolytic release of the coil requires a period of time so that rapid detachment of the coil from the pusher does not occur.

Another technique for detaching an embolic coil uses a mechanical connection between the coil and the pusher. For example, one such device uses interlocking clasps which are secured to each other by a control wire that extends the length of the catheter. Retraction of the control wire uncouples the coil from the pusher.

While mechanical connections between coils and pusher wires allow quick delivery, such systems require additional control mechanisms (i.e., control wires) to deploy the coil. In addition, mechanical connections can suffer from unwanted detachment during delivery through a catheter. Accordingly, while conventional systems provide effective coil delivery, there remains room for improvement.

Patent document n°EP 0829236A discloses engaging members that separate from each other in a passive way, such that when the catheter is not holding them together and/or a control wire is removed, the coil assembly is freed. However, the engaging members only really separate when the insertion cathether with the push wire/second engaging member are moved.

Patent documents n°WO 93/11719A and WO 93/11825A show the use of expendable members that actively separate the engagement members. However, the expandable members are positioned adjacent to the engagement members, which requires additional difficulties in construction and cost.

### SUMMARY OF THE INVENTION

Disclosed herein are methods and systems for delivering an implantable device to a target site using a detachable link. The described systems overcome the drawbacks of conventional mechanical interlocks and provide a more robust coil delivery system that reduces the need to match interlocks with specifically sized catheters. In addition, the systems can reduce the chance of premature device detachment and/or jamming. In one such embodiment, the systems can include a detachable link and a device for inhibiting premature detachment of the detachable link during delivery of the implantable device through a catheter lumen.
An implant delivery system according to the invention is defined in appended claim 1.

This implant delivery system includes an elongate medical device such as a catheter and an implantable medical device mated to a pusher wire via a detachable link comprising first and second engaging members. The first and second engaging members include first surfaces for contacting an inner surface of the catheter and second surfaces for mating with each other. The system further comprises an expandable member adapted to apply a force on the first and second engaging members to inhibit premature detachment or jamming of the system as the system passes through the catheter.

In particular, the expandable member is positioned between the first and second engaging members and adapted to expand and contract to account for variations in the internal diameter of a catheter. In a catheter having a narrow inner lumen the expandable member can compress to allow the system to pass through the catheter. Conversely, in a larger diameter catheter the expandable member can expand the width of the detachable link such that a width of the detachable link matches an internal diameter of the catheter lumen.

In addition, the force applied by the expandable member can inhibit relative movement of the engaging members to prevent premature detachment and/or jamming of the detachable link as the system travels through a catheter. For example, the expandable member can limit relative pivotal, rotation, axial, and/or radial movement of the engaging members.

In one aspect, the expandable member is an elastomer positioned between the second surface of the first engaging member and the second surface of the second engaging member. In another aspect, the expandable member is formed from a sponge material. In yet another aspect, the expandable member is a spring.

### BRIEF DESCRIPTION OF THE DRAWINGS

The accompanying drawings, which are incorporated in and constitute a part of this specification, illustrate illustrative embodiments of the invention and, together with the description, serve to explain the principles of the invention.

FIG. 1 shows a partial view of one embodiment of a system described herein for delivering a detachable, implantable device;

FIG. 2A is a cut-away view of the system of FIG. 1 positioned within a catheter:

FIG. 2B is a perspective view of the detachable, implantable device of FIG. 2A detaching after exiting the distal end of a catheter;

FIG. 3A is a cross-sectional view of another embodiment of a system for delivering a detachable, implantable device;

FIG. 3B is a cross-sectional view of the system of FIG. 3A positioned within a larger diameter catheter;

FIG. 4A is a cross-sectional view of FIG. 3A;

FIG. 4B is a cross-sectional view of FIG. 3B;

FIG. 5A is a side view of an engaging member used with the system described herein;

FIG. 5B is a perspective view of the engaging member of FIG. 5A;

FIG. 6A is a side view of another embodiment of the engaging member shown in FIG. 5A;

FIG. 6B is a perspective view of the engaging member of FIG. 6A;

FIG. 7A is a cross-sectional view of another embodiment of a system for delivery a detachable, implantable device;

FIG. 7B is a cross-sectional view of the system of FIG. 7A positioned within a larger diameter catheter;

FIG. 7C is a perspective view of the system of FIG. 7A delivering a detachable, implantable device;

FIG. 8 is yet another embodiment of a system for delivering a detachable, implantable device;

### DETAILED DESCRIPTION

Disclosed herein are methods and systems for delivering an implantable device to a target site, particularly, a detachable, implantable device. In one aspect, such detachable, implantable devices can be mated to a pusher wire via a detachable link that comprises first and second engaging members. Discussed below are a variety of devices for preventing unwanted detachment of the detachable link during delivery of the detachable, implantable device through a catheter. In one embodiment, a compressible member expands and contracts to account for variations in the internal diameter of the catheter. In another embodiment, a sleeve is placed around at least portion of the engaging members to reduce the chance of unwanted deployment of the detachable device during delivery through the catheter.

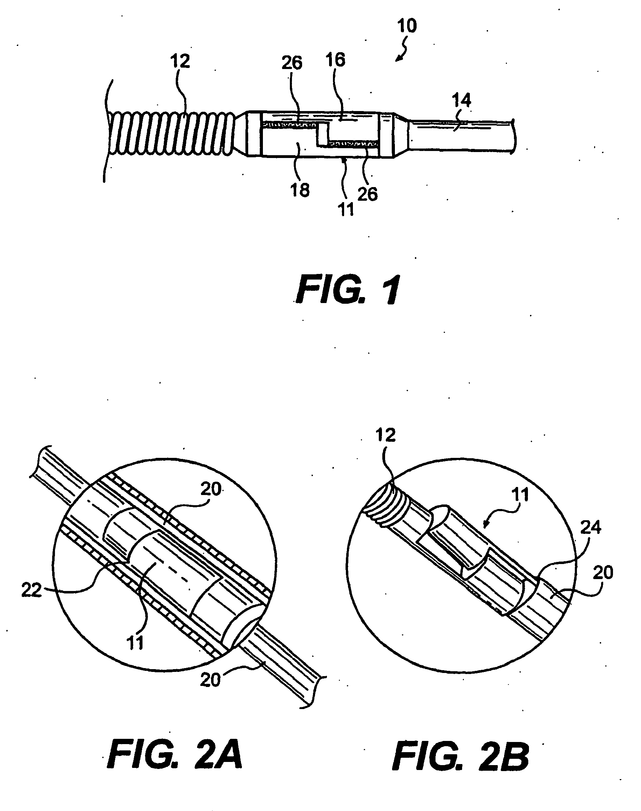

FIG. 1 provides a partial side view of a system 10 for delivering an implantable device, in this case an embolic coil 12 (the terms "coil" and "embolic coil" are used interchangeably herein). The system includes a detachable link 11 and an expandable member 26 for releasably joining the embolic coil 12 and a pusher wire 14. Detachable link 11 can include a first engaging member 16 and a second engaging member 18 that are sized and shaped to mate with one another. As discussed below, engaging members 16, 18 can have a variety of shapes and/or sizes that provide a detachable connection that self detaches after exiting the distal end of a catheter.

One skilled in the art will appreciate that the embolic coil 12 and pusher wire 14 are merely representative of the environment in which detachable link 11 and expandable member 26 operate, and that a variety of alternative medical devices could be substituted. For example, the systems described herein could be used to deliver a variety of implantable devices in addition, or as an alternative, to the embolic coil. Similarly, the pusher wire represents the variety of control devices for moving an implantable device through a lumen of a medical instrument. In addition, as disclosed in co-pending Application Serial No. 11/248,033, entitled "Multiple interlocking Detachable Coils," filed October 12, 2005, and incorporated by reference in its entirety, a second coil, or other such device, could be linked between pusher wire 14 and coil 12 via additional detachable links. Still further, while coil delivery system 10 is generally described with respect to the detachable link traveling through a catheter, one skilled in the art will appreciate that detachable link 11 may travel through a variety of medical instruments, such as, for example, introducers, and that the methods and devices describe herein are equally applicable to any medical device having a lumen for the delivery of a detachable, implantable device. In particular, the term "catheter" as used herein can refer to the variety of medical devices having an inner lumen adapted for receiving a medical instrument and/or implantable device.

FIG. 2A illustrates a partial cutaway view of a catheter 20 containing an exemplary detachable link 11 in the "locked" position. During passage through catheter 20, the inner surface 22 of catheter 20 can prevent detachable link 11 from "unlocking." For example, the diameter/width of the detachable link can closely match the internal diameter of the catheter such that detachable link 11 does not have sufficient room to detach within catheter 20. However, as shown in FIG. 2B, once link 11 reaches the distal end 24 of catheter 20, detachable link 11 is no longer constrained by catheter 20 and can open to allow delivery of coil 12.

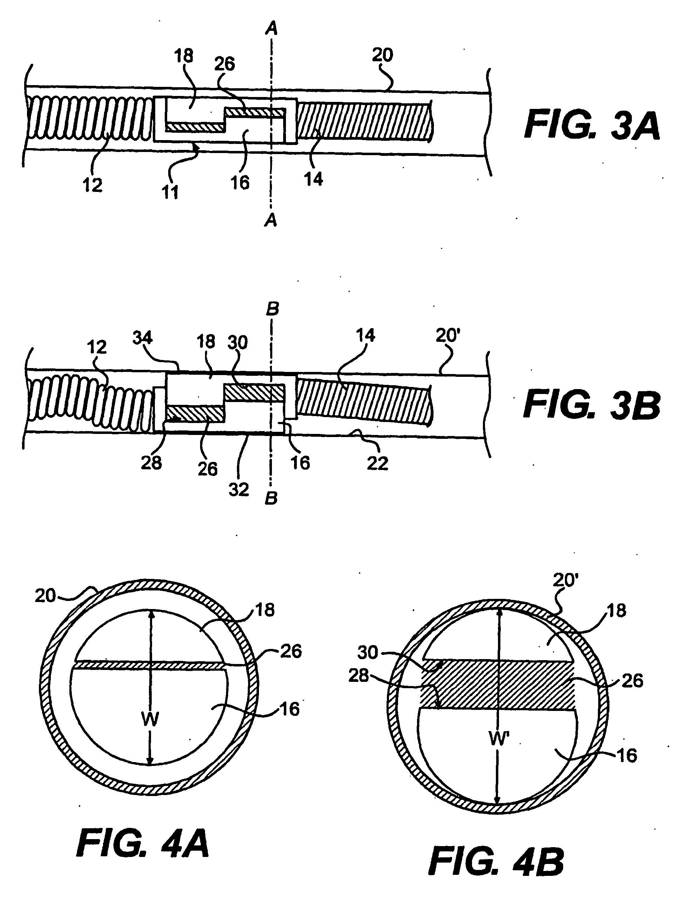

Expandable member 26 allows detachable link 11 to travel through catheters having diameters larger than the outside diameter of the detachable link without detaching or jamming. For example, FIGS. 3A and 3B illustrate coil 12, detachable link 11, and pusher wire 14 in two differently sized catheters 20, 20', respectively. In FIG. 3A the width of the coil delivery system 10 closely matches the internal diameter of catheter 20 with expandable member 26 in a compressed configuration. In FIG. 3B the larger diameter of catheter 20' provides some clearance between the coil/pusher wire and the internal surface of catheter 20'. However, in catheter 20' expandable member 26 has expanded the dimensions (e.g., diameter or a cross-sectional width) of the detachable link to account for the larger diameter of catheter 20'. With expanded member 26 in an expanded configuration there is less room between the outer surface of detachable link 11 and the inner surface of the catheter. The expanded expandable member 26 thereby restricts relative movement between engaging members 16, 18 and help to reduce the chance of detachable link 11 jamming or unlocking prior to delivery of coil 12.

In one embodiment, when expandable member 26 expands, at least a portion of at least one of outer surfaces 32, 34 of engaging members 16, 18 can be pushed against an inner surface 22 of the catheter. Contact between the outer surfaces of the engaging members 32, 34 and the inner surface 22 of the catheter allows detachable link 11 to slide through the catheter but restricts movement of the engaging members relative to the catheter wall, and thereby relative to one another. For example, with engaging members 16, 18 in contact with inner surface 22 of the catheter, the amount of relative pivotal, rotational, axial, and/or radial movement between engaging members 16, 18 can be limited.

FIGS. 4A and 4B provide a cross-sectional view of FIGS. 3A and 3B taken along lines A-A and B-B, respectively. As shown in FIG. 4A, the cross-section of catheter 20 and detachable link 11 are both generally circular, however, both catheter and detachable link could have a variety of different shapes including rectangular, oval, D-shaped, triangular, and/or irregular (however, for the sake of convenience, the dimensions of the cross-section of the catheter will be referred to as a diameter). In addition, the catheter and detachable link could have different cross-sectional shapes from one another. In FIG. 4A the cross-sectional width W of detachable link 11 closely matches the internal diameter of the catheter 20. In addition, although not required, the shape of detachable link 11 also closely matches the internal shape of catheter. In FIG. 4B the diameter of catheter 20' is larger than the diameter of catheter 20 and expandable member 26 has expanded. Expanded expandable member 26 has changed the dimensions of detachable link 11 such that the detachable link has a cross-sectional width W' greater than width W. In one aspect, expandable member 26 has expanded detachable link a sufficient distance to closely match an inside diameter of catheter 20'. In another aspect, detachable link could expand less than the full diameter of catheter 20'.

In addition to assisting with stabilization of detachable link 11 during transit through a catheter, the expandable member can also facilitate detachment of coil 12 after detachable link 11 exits the catheter. Once detachable link has traversed catheter 20 and has been delivered, the detachable link and expandable member are no longer constrained by the catheter walls and can fully expand. The force applied by expandable member 26 on engaging members 16, 18 can help to separate the engaging members and thereby facilitate deployment.

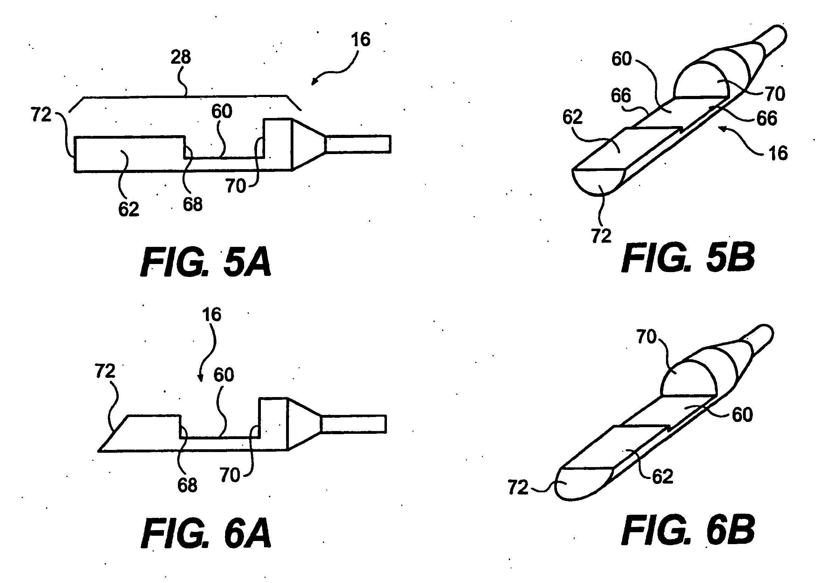

As mentioned above, engaging members 16, 18 can have a variety of configurations adapted to releasably join coil 12 and pusher wire 14. In one aspect, the engaging members are generally configured such that opposed mating surfaces 28, 30 (FIGS. 3B and 4B) reversibly accept a portion of an adjacent engaging member. FIGS. 5A and 5B illustrate an exemplary engaging member (e.g., engaging member 16) having a mating surface 28 comprising a receiving area 60 and a protrusion 62. One skilled in the art will appreciate that protrusion 62 and receiving area 60 can have a variety of shapes including, for example, a circular, oval, rectangular, multi-sided, or irregular shapes that are adapted to mate with receiving areas and protrusions of a corresponding, or different, shape. In the illustrated embodiment, receiving area 60 has open sides 64, 66 and is bounded by end surfaces 68, 70. However, receiving area 60 can alternatively have closed sides.

In use, end surfaces 68, 70 can allow coupled engaging members to push/pull one another. For example, end surface 68 can provide a contact area for pulling on a similar vertical surface on an adjacent engaging member (e.g., engaging member 18). When engaging member 16 is pushed, end surface 70 can be pushed by a surface (e.g., surface 72) on an adjacent engaging member. While, surfaces 68, 70, and 72 are illustrated as vertical, in another embodiment, illustrated in FIGS. 6A and 6B, at least one of the vertical surfaces of engaging members can have a ramped configuration.

Mating surfaces 28, 30 of engaging members 16, 18 can, in one embodiment, mate with expandable member 26. For example, depending on the structure and materials from which expandable member 26 is formed, the expandable member can be adhered, welded, mechanically connected, and/or otherwise fixed to the various surfaces of receiving area 60 and protrusion 62. In addition, expandable member 26 can mate with more than one of the engaging members. For example, the expandable member can be fixedly mated with both mating surfaces 28, 30. Moreover, more than one expandable member can be positioned on mating surfaces 28, 30 and can cover all or a portion of the length and width of mating surfaces 28, 30. In one aspect, mating surfaces 28, 30 can include features to assist with connecting to expandable member 26 including, for example, a recessed area (not illustrated) in which at least a portion of the expandable member is seated.

One skilled in the art will appreciate that the force and distance with which expandable member 26 expands can be varied depending on the force and distance with which one desires expandable member 26 to expand detachable link 11. Greater expansive force can increase the stability of the connection between the engaging members and result in a more robust detachable link. However, as expandable member 26 applies increasing force on engaging members 16, 18, and therefore on the wall 22 of the catheter, the amount of friction which must be overcome to move system 10 through a catheter also increases. As such, the force and distance with which expandable member 26 expands should be chosen such that the detachable link contracts/expands when placed in differently sized catheters, but does not prevent movement of the system through a catheter. One skilled in the art will appreciate that the dimensions and materials used to form expandable member 26 can be varied appropriately.

In general, expandable member 26 can be formed in whole or part by the variety of biocompatible materials having the ability to expand and contract as the detachable link is delivered through any of a variety of medical devices. In one embodiment, expandable member 26 is formed from an elastomer which can be compressed between engaging members 16, 18, and when compressed, will apply a force against the engaging members. Examples of elastomeric materials include various biocompatible materials, such as, for example silicone rubber, urethane based polymers, natural rubber, and combinations thereof. In one exemplary aspect, the elastomeric material can have a hardness in the range of about 75 Shore A and 55 Shore D.

As mentioned above, elastomeric expandable member 26 can be located on and/or mated with at least one of opposing surfaces 28, 30. For example, expandable member 26 could be adhered, mechanically, and/or frictionally engaged with surface 28 and/or 30. In one embodiment, expandable member 26 could be formed from multiple individual portions of elastomeric material positioned between engaging members 16, 18. Alternatively, expandable member 26 is formed from a unitary portion of elastomeric material.

In another embodiment, expandable member 26 can be formed from an absorbent (or adsorbent) material. In a catheter having a smaller diameter (e.g., catheter 20) the absorbent material remains compressed, but can expand in larger diameter catheters and absorb a liquid. When expandable member 26 is in an expanded configuration, the absorbed liquid within expandable member 26 can resist compression. In applications were retrograde blood flow is an issue, the use of absorbent materials can help to stem effluent. Alternatively, or additionally, fluids (such as saline solution) can be added to the catheter to provide fluid for expandable member 26 to expand. Examples of absorbent or adsorbent materials can include biocompatible sponges, adsorbent gels and polymers, ionically active materials, and combinations thereof.

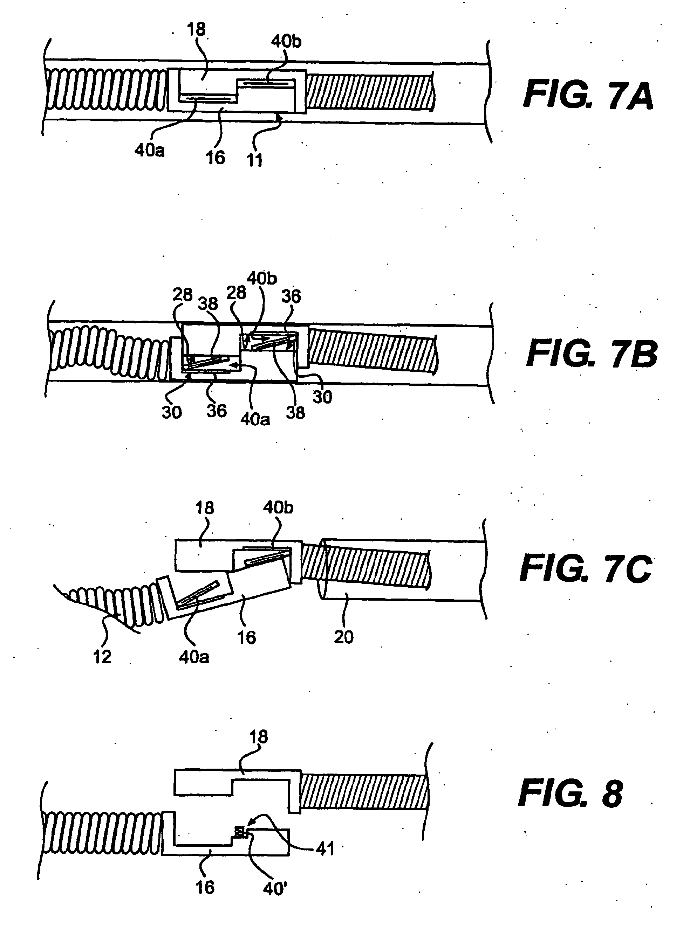

In another embodiment, the expandable member could be formed by a spring. FIGS. 7A through 7C illustrate detachable link 11 with expandable member 26 formed by springs 40a, 40b. As shown, springs 40a, 40b can be leaf-type springs having a first arm 36 mated with a portion of one of the engaging members 16, 18 and a hinged or sprung second arm 38 that applies a force against a surface (e.g., surface 28, 30) of an adjacent engaging member 16, 18 when compressed. One skilled in the art will appreciate that while two springs 40a, 40b are illustrated in FIGS. 7A through 7C, a single spring or more than two springs could be used.

In FIG. 7A, detachable link 11 moves through catheter 20 and expandable member 26 is compressed. When traveling through catheter 20' (FIG. 7B) springs 40a, 40b expand to help prevent jamming or premature detachment of detachable link 11. Once detachable link 11 reaches the distal end 24 of catheter 20, springs 40a, 40b can help to separate the first and second engaging members 16, 18. FIG. 7C illustrates detachment of coil 12 after exiting catheter 20. As shown, the expansive force of springs 40a, 40b pushes engaging members 16, 18 apart to facilitate delivery.

A variety of different types of springs can be used with detachable link 11 including, for example, leaf spring, coiled spring, and combinations thereof. FIG. 8, illustrates a cross-sectional view of a coil springs 40' positioned to apply a force against engaging members 16, 18 when compressed. In one aspect, spring 40' can be positioned in a cavity or recess 41 to provide a low profile expandable member.

Mating of spring 40, 40' with engaging members 16, 18 can be achieved in a variety of ways, including, for example, adhering, mechanically attaching, and/or welding. In addition, one skilled in the art will appreciate that one, two, or more than two springs can be used to stabilize detachable link 11.

Expandable member 26 can be positioned in a variety of locations to assist with stabilizing detachable link 11 as it moves through a catheter. In one embodiment, as shown in FIGS. 1 - 4B and 7A - 8, expandable member 26 is positioned between engaging members 16, 18 such that expansion of expandable member 26 causes engaging members 16, 18 to move away from one another and toward the inner surface 22 of the catheter.

Regardless of the form of the expandable member (e.g., the various expandable members illustrated in FIGS 1 through 8), the amount of force which the expandable member transmits to the walls 22 of the catheter through the engaging members 16, 18 or applies directly on the walls 22 of the catheter will vary depending on the chosen materials and/or structure of the expandable member. In addition, the force applied by the expandable member will vary depending on the amount of compression required (i.e., will vary with the size of the catheter). The variation in force applied results in changes in the amount of friction that must be overcome to move the embolic coil through the catheter. Preferably, the expandable member should not result in excessive friction and cause difficulty in moving and/or controlling the delivery of the coil. As such, one skilled in the art will appreciate that the force applied by the expandable member may need to be adjusted such that premature detachment and/or jamming are minimized while allowing the coil delivery system to move through the catheter with ease. In addition to the properties of the expandable member, other factors can also play a part in ease of coil delivery including the properties of the inner surface of the catheter and the properties of the outer surface of the various elements of the coil delivery system.

While various materials are described above with respect to the disclosed expandable members (e.g., the various expandable members illustrated in FIGS 1 through 8), in one embodiment, the expandable member could have shape memory properties (e.g., formed of nitinol or shape-memory polymer). The shape memory material can be biased toward expansion upon exposure to body temperature. During shipping and/or storage, the detachable link will apply less pressure on packaging. In addition, prior to heating to a body temperature, the detachable link can be easier to transfer from an introducer sheath into a microcatheter.

Other embodiments of the invention will be apparent to those skilled in the art from consideration of the specification and practice of the invention disclosed herein. It is intended that the specification and examples be considered as exemplary only, with a true scope of the invention indicated in the appended claims.

## Claims

1. An implant delivery system (10), comprising:
an elongate medical device (20) having an inner lumen;
an implantable medical device mated to a pusher wire (14) via a detachable link (11) comprising first and second engaging members (16, 18);
the first engaging member (16) including a first surface (32) for contacting an inner surface (22) of said inner lumen and a second surface (28) for mating with the second engaging member (18); and
the second engaging member (18) having a first surface (34) for contacting the inner surface (22) of said inner lumen and a second surface (30) for mating with the first engaging member;
the implant delivery system being **characterized in that** it further comprises an expandable member (26) positioned between the first and second engaging members, the expandable member being adapted to press the first surface (32) of the first engaging member (16) and the first surface (34) of the second engaging member (18) against the inner surface (22) of said inner lumen during delivery of the implantable medical device.

2. The system of claim 1, wherein the expandable member (26) is adapted to expand and contract to vary the width of the detachable link (11) such that a width of the detachable link matches an internal diameter of said inner lumen.

3. The system of claim 1 or 2, wherein the expandable member (26) is an elastomer positioned between the second surface (28) of the first engaging member (16) and the second surface (30) of the second engaging member (18).

4. The system of claim 1 or 2, wherein the expandable member (26) is formed from a sponge material.

5. The system of claim 1 or 2, wherein the expandable member (26) is a spring (40a, 40b).

6. The system of any one of claims 1 to 5, wherein the detachable link (11) has a shape that substantially corresponds to the shape of said inner lumen when the expandable member (26) is compressed.

7. The system of claim 1 or 2, wherein the second surface (28, 30) of at least one of the first and second engaging members includes a mating feature for mating with the expandable member (26).

8. The system of claim 7, wherein the mating feature is a recess for receiving at least a portion of the expandable member (26).

## Patentansprüche

1. Implantat-Zuführungssystem (10), umfassend:
eine längliche medizinische Vorrichtung (20), die ein inneres Lumen hat,
eine implantierbare medizinische Vorrichtung, die über eine lösbare Verbindung (11), die ein erstes und ein zweites Eingriffselement (16, 18) umfaßt, an einen Schubdraht (14) gepaßt ist,
wobei das erste Eingriffselement (16) eine erste Fläche (32) zum Berühren einer Innenfläche (22) des inneren Lumens und eine zweite Fläche (28) zum Zusammenpassen mit dem zweiten Eingriffselement (18) aufweist und
wobei das zweite Eingriffselement (18) eine erste Fläche (34) zum Berühren der Innenfläche (22) des inneren Lumens und eine zweite Fläche (30) zum Zusammenpassen mit dem ersten Eingriffselement hat,
wobei das Implantat-Zuführungssystem **dadurch gekennzeichnet ist, daß** es ferner ein ausdehnbares Element (26) umfaßt, das zwischen dem ersten und dem zweiten Eingriffselement angeordnet ist, wobei das ausdehnbare Element dazu ausgebildet ist, die erste Fläche (32) des ersten Eingriffselements (16) und die erste Fläche (34) des zweiten Eingriffselements (18) während des Zuführens der implantierbaren medizinischen Vorrichtung gegen die Innenfläche (22) des inneren Lumens zu pressen.

2. System nach Anspruch 1, wobei das ausdehnbare Element (26) dazu ausgebildet ist, sich auszudehnen und zusammenzuziehen, um die Breite der lösbaren Verbindung (11) derart zu verändern, daß sich die Breite der lösbaren Verbindung einem Innendurchmesser des inneren Lumens anpaßt.

3. System nach Anspruch 1 oder 2, wobei das ausdehnbare Element (26) ein Elastomer ist, das zwischen der zweiten Fläche (28) des ersten Eingriffselements (16) und der zweiten Fläche (30) des zweiten Eingriffselements (18) angeordnet ist.

4. System nach Anspruch 1 oder 2, wobei das ausdehnbare Element (26) aus einem Schwammwerkstoff geformt ist.

5. System nach Anspruch 1 der 2, wobei das ausdehnbare Element (26) eine Feder (40a, 40b) ist.

6. System nach einem der Ansprüche 1 bis 5, wobei die lösbare Verbindung (11) eine Form hat, die im Wesentlichen der Form des inneren Lumens entspricht, wenn das ausdehnbare Element (26) zusammengedrückt ist.

7. System nach Anspruch 1 oder 2, wobei die zweite Fläche (28, 30) wenigstens eines von dem ersten und dem zweiten Eingriffselement ein Paßmerkmal zum Zusammenpassen mit dem ausdehnbaren Element (26) aufweist.

8. System nach Anspruch 7, wobei das Paßmerkmal eine Aussparung zum Aufnehmen wenigstens eines Abschnitts des ausdehnbaren Elements (26) ist.

## Revendications

1. Système pour délivreur un implant (10), comportant :
un dispositif médical allongé (20) ayant une lumière intérieure ;
un dispositif médical implantable associé à un câble de poussée (14) via une liaison détachable (11) comprenant des premier et deuxième éléments d'engagement (16, 18);
le premier élément d'engagement (16) comprenant une première surface (32) de contact avec une surface intérieure (22) de ladite lumière intérieure et une deuxième surface (28) de connexion avec le deuxième élément d'engagement (18) ; et
le deuxième élément d'engagement (18) ayant une première surface (34) de contact avec la surface intérieure (22) de ladite lumière intérieure et une deuxième surface (30) de connexion avec le premier élément d'engagement ;
le système pour délivrer un implant étant **caractérisé en ce qu'**il comporte en outre un élément expansible (26) positionné entre les premier et deuxième éléments d'engagement, l'élément expansible étant adapté pour presser la première surface (32) du premier élément d'engagement (16) et la première surface (34) du deuxième élément d'engagement (18) contre la surface intérieure (22) de ladite lumière intérieure lorsque le dispositif médical implantable est délivré.

2. Système selon la revendication 1, dans lequel l'élément expansible (26) est adapté pour s'expanser et se contracter afin de faire varier la largeur de la liaison détachable (11) de telle sorte qu'une largeur de la liaison détachable correspond à un diamètre interne de ladite lumière intérieure.

3. Système selon la revendication 1 ou 2, dans lequel l'élément expansible (26) est un élastomère positionné entre la deuxième surface (28) du premier élément d'engagement (16) et la deuxième surface (30) du deuxième élément d'engagement (18).

4. Système selon la revendication 1 ou 2, dans lequel l'élément expansible (26) est formé à partir d'une matière spongieuse.

5. Système selon la revendication 1 ou 2, dans lequel l'élément expansible (26) est un ressort (40a, 40b).

6. Système selon l'une quelconque des revendications 1 à 5, dans lequel la liaison détachable (11) a une forme qui correspond sensiblement à la forme de ladite lumière intérieure quand l'élément expansible (26) est comprimé.

7. Système selon la revendication 1 ou 2, dans lequel la deuxième surfaces (28, 30) d'au moines un des premier et deuxième éléments d'engagement comprend une caractéristique de correspondante pour la connexion avec l'élément expansible (26).

8. Système selon la revendication 7, dans lequel la caractéristique de correspondante est un renfoncement pour recevoir au soins une partie de l'élément expansible (26).
